# FASCICULE DE BREVET EUROPEEN

(11) **EP 1 226 835 B1**
(45) Date de publication et mention de la délivrance du brevet: **22.09.2010**
(21) Numéro de dépôt: 02290150.8
(22) Date de dépôt: 21.01.2002
(51) Int. Cl.: A61L 9/14, A61L 9/01

(54) **Procédé de désinfection de locaux par thermonébulisation d'une solution aqueuse d'acide peracétique**
Desinfektionsverfahren für Räume mittels Thermo-Vernebelung einer wässrigen Peressigsäurelösung
Method of disinfecting premises by thermospraying an aqueous solution of peracetic acid

(30) Priorité: 26.01.2001 FR 0101086
(43) Date de publication de la demande: 31.07.2002
(73) Titulaire: Bioxal, 75007 Paris Cedex 07 (FR)
(72) Inventeur: Le Rouzic, Daniel, 95120 Ermont (FR); Gamet, Jean-Claude, 71460 Saint Martin du Tartre (FR)
(74) Mandataire: Conan, Philippe Claude

(56) Documents cités:
- EP-A- 1 068 873
- EP-A- 1 070 505
- FR-A- 2 759 911
- FR-A- 2 796 286
- GB-A- 1 570 492
- US-B1- 6 168 808

## Description

L'invention se rapporte à un nouveau procédé de désinfection de locaux et à la composition mise en oeuvre.

La bio-sécurité des produits alimentaires exigée par les consommateurs, implique une prise de responsabilité plus importante de la filière alimentaire et plus particulièrement des éleveurs, à la qualité et à l'innocuité des viandes mises à la consommation humaine. Il en découle l'obligation impérieuse d'élever les animaux et de produire la viande dans d'excellentes conditions sanitaires et aussi d'être capable d'en apporter la preuve. Les conditions d'hygiène dans lesquelles opère la filière viande sont donc essentielles pour atteindre cet objectif.

C'est pourquoi, les procédures de nettoyage et de désinfection des locaux d'élevage s'inscrivent dans la stratégie globale de l'hygiène de la filière. Ces procédures comprennent notamment les étapes suivantes :
(a) - La préparation des bâtiments comme le dégagement de tous leurs accès ou la vidange des pré-fosses,
(b) - le nettoyage des surfaces à l'eau chaude avec un nettoyant - dégraissant,
(c) - la désinfection primaire des surfaces avec un désinfectant à large spectre,
(d) - la désinfection secondaire ou terminale des surfaces par sublimation de formol ou aérolisation d'un désinfectant et
(e) - le contrôle bactériologique.

Ces procédures permettent ainsi d'assainir les surfaces tout en quantifiant les résultats obtenus à l'issue de chaque étape. Généralement, le nettoyage diminue 10 fois la population bactérienne, la désinfection primaire réduit de 10³ fois, le nombre de germes restant après le nettoyage et la désinfection secondaire réduit de 10 fois le nombre de germes survivants.

Actuellement, trois techniques sont retenues pour la désinfection secondaire :
(a) - La sublimation du formol qui est un désinfectant très actif mais d'un emploi très délicat. Les locaux doivent être totalement hermétiques et le traitement doit durer 12 heures. De plus le délai de récupération est trop long car il faut aérer longtemps les locaux pour atteindre la valeur limite d'exposition aux vapeurs (VLE) ;
(b) - L'aérolisation d'un désinfectant qui consiste en la production d'un brouillard de fines particules de désinfectant de taille de l'ordre de 10 à 30 microns. Cette méthode est difficile à mettre en oeuvre pour obtenir un brouillard stable et homogène dans tout le local. Les produits désinfectants nécessitent trois à quatre heures de contacts ;
(c) - La thermonébulisation d'un désinfectant consistant en la production d'un brouillard de très fines particules de désinfectant de taille de l'ordre de 0,5 à 1 micron. On obtient par cette méthode une dispersion rapide et homogène du désinfectant. Cette dernière méthode est devenue le procédé de référence en matière de désinfection secondaire.

Cependant, lorsque cette méthode est mise en oeuvre avec des désinfectants aldéhydiques tels que le glutaraldéhyde, le temps de contact nécessaire est toujours supérieur à deux heures et il se produit une polymérisation du composé, qui conduit peu à peu à l'encrassement du thermonébulisateur. D'autre part comme, pour les méthodes précédentes, les locaux doivent rester inoccupés pendant 12 heures pour atteindre la valeur limite d'exposition aux vapeurs (VLE).

La demanderesse a donc cherché à mettre au point un nouveau procédé de désinfection par thermonébulisation, qui n'ait pas les inconvénients exposés ci-dessus tant en termes d'encrassage du thermonébulisateur que de VLE.

Le document US 6,168,808 B1 décrit un procédé de désinfection comprenant la nébulisation d'une solution aqueuse d'acide peracetique, la solution comprenant entre autre un agent tensionactif non-ionique et un oxyde d'amine.

C'est pourquoi l'invention a pour objet, un procédé désinfection d'un local, tel que défini à la revendication 1.

Par étape de lavage, on entend dans le cadre de la présente invention, étape de lavage à l'eau chaude ou avec une solution aqueuse d'un ou plusieurs détergents, suivie si désiré ou si nécessaire, d'une ou plusieurs étapes de rinçage à l'eau chaude ou à l'eau froide.

Le procédé tel que défini ci-dessus est mis en oeuvre de façon à thermonébuliser une quantité d'acide peracétique de préférence entre 50 ppm et 100 ppm par mètre cube de local à désinfecter.

La température de thermonébulisation est généralement comprise entre 0°C et 45°C et est de préférence comprise entre 25°C et 35°C.

Dans le procédé tel que défini ci-dessus, La dilution de ladite solution d'acide peracétique est effectuée manuellement avant mise en oeuvre de l'appareil à thermonébuliser. On utilise des solutions plus ou moins concentrées en acide peracétique.

Comme agent tensioacif non-ionique approprié à la présente invention, il y a notamment les composés commerciaux suivants :

| nom | composition chimique |
|---|---|
| Génapol™ 2822 | mélange d'alcools alkoxylés en C₁₀, C₁₂, C₁₄, (5 OE, 4 OP) |
| Génapol™ 2908D | mélange d'alcools alkoxylés en C₁₁, C₁₃, C₁₅, (6 à 7 OE, 3 OP) |
| Génapol™ 2909 | mélange d'alcools alkoxylés en C₁₂ et C₁₄, (5 OE, 3 OP) |
| Simulsol™ NW 900 | mélange d'alcools alkoxylés (x OE, y OP) |

Dans la définition de la formule (II) telle que définie précédemment, le groupe - [CH(R₅)-CH(R₆)]ₙ- peut représenter, soit une chaîne polyéthoxylée (R₅ et R₆ = H] soit une chaîne polypropoxylée (l'un des R₅ ou R₆ = H et l'autre est un radical méthyle), soit une chaîne polybutoxylée (R₅ et R₆ = CH₃], soit une chaînes constituée d'un mélange de deux sortes ou des trois sortes de groupes, éthoxyle, propoxyles ou butoxyles, les différents fragments étant distribués au sein de ladite chaîne, de manière séquencée ou aléatoire.

Les inhibiteurs de corrosion, les agents stabilisants et les catalyseurs acides sont ceux habituellement présents dans les solutions aqueuses d'acides peracétique.

L'exemple suivant illustre l'invention sans toutefois la limiter.

### Exemple d'application :

Après nettoyage et désinfection primaire d'un local d'élevage, on thermonébulise 6 ml / m³ de local d'une solution aqueuse préparée par dilution au tiers dans l'eau de ville d'une composition contenant 5,4 % en poids d'acide peracétique, 14,5 % de peroxyde d'hydrogène, 17,9 % d'acide acétique, 0,2 % d'agent tensioactif non ionique (Génapol™ 2908 D) 0,7 % d'inhibiteur de corrosion (dihydrogénophosphate de sodium), 0,1 % d'oxyde d'amine (Aromox™ MCD-W), 0,2 % d'acide hydroxy-éthylidène di phosponique,(DEQUEST™ 2010), 0,4 % d'acide sulfurique et eau qsp 100 %, au moyen d'un thermonébulisateur **TF 35 EL** commercialisé par la société **IGEBA.**

On laisse ensuite le local au repos pendant une heure puis on le ventile pendant quinze minutes avant réutilisation.

L'analyse des prélèvements effectués avant la ventilation finale selon la norme française NFT 72281, permet de mettre en évidence un abattement supérieur à 5 log en terme de bactéricidie, supérieur à 4 log en terme de fungicidie et supérieur à 3 log en terme de sporicidie.

On constate que la composition essayée n'a pas d'équivalent connue en termes d'efficacité désinfectante globale par voie aérienne. D'autre part à plusieurs essais, on ne constate aucune détérioration de l'appareil pouvant être préjudiciables aux opérations de thermonébulisation ultérieures.

## Revendications

1. - Procédé de nettoyage et de désinfection d'un local comprenant au moins une étape de lavage, suivie d'une étape de désinfection primaire, elle-même suivie d'une étape de désinfection secondaire, **caractérisé :**
- **En ce que** l'étape de désinfection secondaire est réalisée par thermonébulisation d'une solution aqueuse d'acide peracétique la thermonébulisation consistant en la production d'un brouillard de très fines particules de taille de l'ordre de 0,5 à 1 micron;
- **En ce que** la quantité d'acide peracétique thermonébulisée est comprise entre 50 ppm et 300ppm par mètre cube de local à désinfecter ;
- **En ce que** ladite solution aqueuse d'acide peracétique comprend :
- Entre 3% et 6% en poids d'acide peracétique,
- Entre 10% et 20 % en poids de peroxyde d'hydrogène, entre 10% et 20% en poids d'acide acétique,
- Entre 0,1 % et 1% en poids d'un composé de formule (II) :
R₄-O-[CH(R₅)-CH(R₆)]ₙ-OR₇ (II),
dans laquelle R4 représente un radical aliphatique linéaire ou ramifié saturé ou insaturé, comportant de 10 à 18 atomes de carbone, et R5 et R6 identiques ou différents représentent indépendamment l'un de l'autre un atome d'hydrogène ou un radical méthyle, R7 représente un atome d'hydrogène, un radical alkyle linéaire ou ramifié, compor tant de un à quatre atomes de carbone ou un radical benzyle et n représente un nombre supérieur ou égal à 1 et inférieur à 20 ;
- Entre 0,05% et 0,5% d'un composé choisi parmi l'oxyde de cocodi méthylamine, l'oxyde de myristamine, l'oxyde de di(hydroxyéthyl) co coamine, l'oxyde de diméthyl stéarylamine, ou l'oxyde de diéthyl stéarylamine ;
- Entre 0,1% et 0,5% en poids d'agent stabilisant ;
- Entre 0,5% et 1% d'inhibiteur de corrosion et/ou entre 0% et 1 % d'acide fort ;
- **En ce que** ladite solution d'acide peracétique est prélablement dilué au tiers avec de l'eau puis thermonébulisée ; et
**En ce que** ledit local est un local d'élevage d'animaux.

2. - Procédé tel que défini à la revendication 1, dans lequel la quantité d'acide peracétique thermonébulisée est comprise entre 50 ppm et 100 ppm par mètre cube de local à désinfecter.

3. . Procédé tel que défini à l'une des revendications 1 ou 2, **caractérisé en ce que** ledit local est un local d'élevage avicole.

## Claims

1. Method of cleaning and disinfecting a premises, comprising at least one washing step, followed by a primary disinfecting step, itself followed by a secondary disinfecting step, **characterized:**
- **in that** the secondary disinfecting step is carried out by thermal fogging of an aqueous solution of peracetic acid, the thermal fogging consisting of the production of a fog of very fine particles having a size of the order of 0.5 to 1 micron;
- **in that** the amount of peracetic acid subjected to thermal fogging is between 50 ppm and 300 ppm per cubic metre of premises to be disinfected;
- **in that** said aqueous solution of peracetic acid comprises:
- between 3% and 6% by weight of peracetic acid,
- between 10% and 20% by weight of hydrogen peroxide, between 10% and 20% by weight of acetic acid,
- between 0.1% and 1% by weight of a compound of formula (II):
R₄-O- [CH(R₅)-CH(R₆)]ₙ-OR₇ (II),
in which R4 represents a saturated or unsaturated, linear or branched aliphatic radical containing from 10 to 18 carbon atoms, and R5 and R6, which may be identical or different, represent, independently of one another, a hydrogen atom or a methyl radical,
R7 represents a hydrogen atom, a linear or branched alkyl radical containing from one to four carbon atoms or a benzyl radical, and n represents a number greater than or equal to 1 and less than 20;
- between 0.05% and 0.5% of a compound chosen from cocodimethylamine oxide, myristamine oxide, di(hydroxyethyl) cocoamine oxide, dimethylstearylamine oxide or diethylstearyl-amine oxide;
- between 0.1% and 0.5% by weight of stabilizer;
- between 0.5% and 1% of corrosion inhibitor and/or between 0% and 1% of strong acid;
- **in that** said solution of peracetic acid is prediluted by a third with water and then subjected to thermal fogging; and
- **in that** said premises is a livestock farming premises.

2. Method as defined in Claim 1, in which the amount of peracetic acid subjected to thermal fogging is between 50 ppm and 100 ppm per cubic metre of premises to be disinfected.

3. Method as defined in either -of Claims 1 and 2, **characterized in that** said premises is a poultry farming premises.

## Patentansprüche

1. Verfahren zur Reinigung und Desinfektion einer Räumlichkeit, das mindestens einen Waschschritt umfasst, gefolgt von einem primären Desinfektionsschritt, ebenfalls gefolgt von einem sekundären Desinfektionsschritt, **dadurch gekennzeichnet:**
- **dass** der sekundäre Desinfektionsschritt durch thermische Vernebelung einer wässrigen Peressigsäure durchgeführt wird, wobei die thermische Vernebelung in der Herstellung eines Nebels aus sehr feinen Teilchen mit einer Größe im Bereich von 0,5 bis 1 Mikron besteht;
- **dass** die Menge der thermisch vernebelten Peressigsäure zwischen 50 ppm und 300 ppm pro Kubikmeter der zu desinfizierenden Räumlichkeit liegt;
- **dass** die wässrige Peressigsäurelösung Folgendes umfasst:
- zwischen 3 Gew.-% und 6 Gew.-% Peressigsäure,
- zwischen 10 Gew.-% und 20 Gew.-% Wasserstoffperoxid, zwischen 10 Gew.-% und 20 Gew.-% Essigsäure,
- zwischen 0,1 Gew.-% und 1 Gew.-% einer Verbindung der Formel (II):
R₄-O-[CH(R₅)-CH(R₆)]ₙ-OR₇ (II),
in der R4 für einen gesättigten oder ungesättigten, linearen oder verzweigten aliphatischen Rest steht, der 10 bis 18 Kohlenstoffatome umfasst, und R5 und R6, die gleich oder verschieden sind, unabhängig voneinander für ein Wasserstoffatom oder einen Methylrest stehen, R7 für ein Wasserstoffatom, einen linearen oder verzweigten Alkylrest, der ein bis vier Kohlenstoffatome umfasst, oder einen Benzylrest steht und n für ein Zahl steht, die größer gleich 1 und kleiner als 20 ist;
- zwischen 0,05 % und 0,5 % einer Verbindung, die aus Kokosdimethylaminoxid, Myristaminoxid, Di(hydroxyethyl)kokosaminoxid, Dimethylstearylaminoxid oder Diethylstearylaminoxid ausgewählt ist;
- zwischen 0,1 Gew.-% und 0,5 Gew.-% eines Stabilisierungsmittels;
- zwischen 0,5 % und 1 % eines Korrosionshemmers und/oder zwischen 0 % und 1 % einer starken Säure;
- **dass** die Peressigsäurelösung drittens vorher mit Wasser verdünnt wird und dann thermisch vernebelt wird und
- **dass** es sich bei der Räumlichkeit um eine Räumlichkeit zur Tierzucht handelt.

2. Verfahren nach Anspruch 1, in dem die Menge der thermisch vernebelten Peressigsäure zwischen 50 ppm und 100 ppm pro Kubikmeter der zu desinfizierenden Räumlichkeit liegt.

3. Verfahren nach einem der Ansprüche 1 oder 2, **dadurch gekennzeichnet, dass** es sich bei der Räumlichkeit um eine Räumlichkeit zur Geflügelzucht handelt.
